Europäisches Patentamt

European Patent Office

Office européen des brevets

(19)

(11) Publication number: **0 319 964 B1**

# EUROPEAN PATENT SPECIFICATION

(12)

(45) Date of publication of patent specification: **18.03.92**   (51) Int. Cl.⁵: **A61K  47/00**, A61K 31/27

(21) Application number: **88120480.4**

(22) Date of filing: **07.12.88**

(54) **Film-formation-type antifungal preparation.**

(30) Priority: **08.12.87 JP 310480/87**

(43) Date of publication of application:
**14.06.89 Bulletin  89/24**

(45) Publication of the grant of the patent:
**18.03.92 Bulletin  92/12**

(84) Designated Contracting States:
**AT BE CH DE ES FR GB GR IT LI LU NL SE**

(56) References cited:
**GB-A- 1 186 691**

**CHEMICAL ABSTRACTS, vol. 105, no. 6, 11th
August 1986, page 396, abstract no. 49073b,
Columbus, Ohio, US; & JP-A-61 69 721 (KAO
CORP.) 10-04-1986**

**CHEMICAL ABSTRACTS, vol. 99, no. 14, 3rd
October 1983, page 344, abstract no.
110789r, Columbus, Ohio, US; & JP-A-58 105
915 (LION CORP.) 24-06-1983**

(73) Proprietor: **SHIONOGI SEIYAKU KABUSHIKI
KAISHA
12, Dosho-machi, 3-chome Higashi-ku,
Osaka-shi
Osaka 541(JP)**

(72) Inventor: **Saitoh, Izumi
1-2-23-601, Kamikoshien
Nishinomiya-shi Hyogo(JP)**
Inventor: **Ikeda, Kaori
2-3-2-706, Takakuradai
Sakai-shi Osaka(JP)**
Inventor: **Doi, Yoshi
10-10, Wakazono-cho
Ibaraki-shi Osaka(JP)**
Inventor: **Egawa, Shohei
2-4-15, Nanatsumatsu-cho
Amagasaki-shi Hyogo(JP)**

(74) Representative: **Vossius & Partner
Siebertstrasse 4 P.O. Box 86 07 67
W-8000 München 86(DE)**

Rank Xerox (UK) Business Services

**Description**

The present invention relates to preparations for topical application which contain tolnaphtate (hereinafter abbreviated as TOL) known as an antifungal agent. More specifically, the preparations of this invention are so designed as to form a flexible, strong, transparent film on the skin and gradually release TOL therefrom. Furthermore, the present invention relates to a process for the production of such preparations.

So far, tinctures, creams, gels or the like preparations have been used for the topical treatment of mycosis.

TOL has a potent action against fungi, especially against *Trychophytons* and, therefore, it has long been used for the treatment of tinea pedis. However, in many cases, mycoses such as athlete's foot primarily occur at moist parts of the body. So when an ointment or a gel preparation is applied to the affected part, it makes the affected part even more moist, thereby causing a strange sensation or staining the clothes. These are the shortcomings of using ointments or gel preparations. A tincture has shortcomings such as taking longer to dry on the applied parts. Furthermore, another shortcoming is that in conventional TOL-preparations TOL crystallizes upon storage or upon application because of its low solubility in most solvents.

The present invention provides film-forming antifugal preparations for topical application, which comprise 0.1% to 1.5% of tolnaphtate, 10% to 20% of a dimethylaminoethyl methacrylate-methacrylic acid ester copolymer (hereinafter abbreviated as DMMA-MA), and 0.5% to 10% of a medium chain fatty acid ester in an alcoholic solvent but containing practically no water.

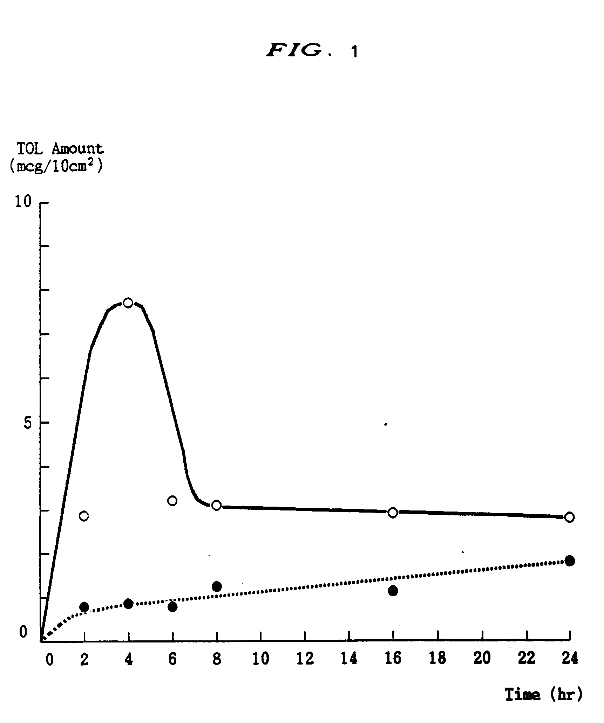

In Fig. 1 the ordinate shows amounts of TOL in the skin, while the abscissa shows the time. The remaining amounts of TOL in the skin are shown by the mark "●" for the control and by the mark "○" for the Example 1 preparation.

The problem underlying the present invention is to find such base ingredients which prevent TOL from crystallising upon storage or after application and to enhance the transdermal absorbability of TOL.
The preparations of this invention enhance and sustain the action of TOL and, therefore, they are expected to be of excellent efficacy in a once-a-day application.

The preparations of this invention are capable of forming a flexible, strong film on the skin when applied and prolong the drug effect. Proportions used in this invention are shown as percentage by weight (w/w%) of certain additives to the total weight of the whole preparation.

This invention can be achieved by dissolving 0.1% - 1.5% of TOL, 10% - 20% of DMMA-MA, and 0.5% - 10% of a medium chain fatty acid ester in an alcoholic solvent. If necessary, 0.1% - 2.5% of a thickening agent and/or a plasticizer may further be added.

TOL used in this invention is a potent antifugal agent and is very popular as a cream- or gel-preparation. Eudragit® E100 is a suitable example of DMMA-MA.

The medium chain fatty acid ester includes glyceryl monocaprate (GMC), tetraglyceryl monocaprate (TGMC), propyleneglycol dicaprate (PGDC), and tetraglyceryl hexacaprate (TGHC). TGMC is especially preferred.

The alcoholic solvent used in this invention is a lower alkanol which is substantially free of water. The lower alkanol includes ethanol, propanol and isopropanol.

Thickening agents including cellulose derivatives such as ethyl cellulose (EC), hydroxypropyl cellulose (HPC) and hydroxypropylmethyl cellulose (HPMC) are preferably used. Plasticizers including propylene glycol (PG) and polyethylene glycol (PEG) are preferably used.

The preparations for tinea pedis of this invention are remarkably improved in that they enhance the transdermal absorbability of TOL and prolong drug effectiveness compared to conventional preparations, creams, gels, tinctures or the like. It can thus be expected that an once-a-day application of the preparation is enough to attain good efficacy. The present invention is explained in more detail in the following Examples and Experiments, which are not intended to limit the scope of the invention.

EXAMPLE 1

Isopropanol (76g) and 5g of TGMC were put in a closed type vessel equipped with a stirrer. DMMA-MA (15g; EUDRAGIT® E100) and 3g of EC were gradually added to give a clear solution, into which 1g of TOL was dissolved while stirring to make the objective preparation (100g) of this invention.

EXAMPLEs 2 - 4

In substantially the same manner as in Example 1, the following instant preparations for tinea pedis were obtained.

Table 1

| Example Components | 2 (%) | 3 (%) | 4 (%) | Control (%) |
|---|---|---|---|---|
| TOL | 1 | 1 | 1 | 1 |
| DMMA-MA | 15 | 15 | 15 | 15 |
| GMC | 5 | | | |
| TGMC | | 5 | | |
| TGHC | | | 5 | |
| PG | | | | 3 |
| Ethanol | | 79 | 79 | 81 |
| Isopropanol | 79 | | | |

EXPERIMENT 1

The precipitation of crystals was examined to study the shelf life stability of each of the following preparations. The change in solution and the presence of crystals were examined by observation with the naked eye or under a microscope.

(Preparations Examined)

Preparations manufactured in Examples 1 to 4
Control: Control preparation shown in Table 1

Table 2

| Preparation | Stored at 5°C in tightly closed container | | | | | |
|---|---|---|---|---|---|---|
| | Initial | After 1 Week | After 2 Weeks | After 1 Month | After 2 Months | After 3 Months |
| 1 | - | - | - | - | - | - |
| 2 | - | - | - | - | - | ○ |
| 3 | - | - | - | - | - | - |
| 4 | - | - | - | - | - | - |
| Control | - | - | ○ | | | |

(Remarks)

-: No change in solution & no formation of crystals were observed.
○: Change in solution & formation of crystals were observed.

EXPERIMENT 2

The *in vivo* transdermal absorption study shown in the following experiments were carried out, basically according to the undermentioned method:

(Test Method)

1. The abdominal hair of male Wistar rats (9 weeks of age , n = 5-8) anesthetised with urethane is

EP 0 319 964 B1

removed carefully with electric clippers and an electric razor.

2. One of these rats is immobilized on its back and then an absorption chamber (application area: 10 cm$^2$) is fixed onto the surface of the hairless abdomen with an instant adhesive.

3. A pre-fixed dose of test material (2 mg as TOL per rat) is placed into the chamber.

4. After a certain period of time, the coating film formed on the skin in the chamber is removed with distilled water and collected into a suitable vessel.

5. The chamber is removed and the application area of the skin is cut off.

6. The sample in Item 4 and the piece of the skin in Item 5 are employed for the TOL content measurement by HPLC.

COMPARATIVE EXPERIMENT 1

Along with the test method mentioned above, comparative studies for transdermal absorption of TOL were carried out on some instant preparations and commercially available ones . Amounts of TOL in the skin 4 hours after the application were as follows.

(Result)

Table 3

| Preparation | Amount of TOL in the Skin (mcg/10cm$^2$) |
|---|---|
| Example 1 | 9.04±2.50 |
| Example 2 | 3.97±1.08 |
| Example 3 | 8.84±2.85 |
| Example 4 | 6.21 |
| Commercially Available | 0.54±0.06 |
| (Remarks) | |
| Commercially Available: Pasca® Gel (by Shionogi & Co., Ltd., containing 1% of TOL) | |

The instant preparations of this invention exhibit much higher transdermal absorbabilities than the (commercially available) gel preparation which is generally believed to exhibit high absorbability.

EXPERIMENT 3

Amounts of TOL in the skin were measured at various times for the preparation of Example 1 and the commercially available preparation (1% Pasca® Gel: made by Shionogi & Co., Ltd.), to evaluate the sustained release of TOL

(Results)

Results are shown in the drawing. As compared with the control, the preparations of this invention have remarkably improved the transdermal absorbability of TOL and, additionally, they are capable of keeping high TOL concentrations in the skin for a long period of time. Consequently, the preparations of this invention have a much larger area under the curve (AUC), which demonstrates a high bioavailability of TOL.

From these results, it is expected that the preparations for tinea pedis of this invention will be of excellent efficacy as once-a-day applications.

**Claims**
**Claims for the following Contracting States : AT, BE, CH, DE, FR, GB, IT, LI, LU, NL, SE**

1. A film-forming antifungal preparation which comprises 0.1% to 1.5% of tolnaphtate, 10% to 20% of a dimethylaminoethyl methacrylate -methacrylic acid ester copolymer, and 0.5% to 10% of a medium chain fatty acid ester in an alcoholic solvent but containing practically no water.

4

EP 0 319 964 B1

2. The film-forming antifungal preparation claimed in Claim 1, wherein said medium chain fatty acid ester is tetraglyceryl monocaprate.

3. The film-forming antifungal preparation claimed in Claim 1, further containing 0.1% to 5% of a thickening agent.

4. The film-forming antifungal preparation claimed in Claim 1, wherein said alcoholic solvent is ethanol or isopropanol.

5. The film-forming antifungal preparation claimed in Claim 3, wherein said thickening agent is ethyl cellulose, hydroxypropyl cellulose, or hydroxypropylmethyl cellulose.

6. A method for preparing a film-formation-type antifungal preparation which comprises combining 0.1% to 1.5% of tolnaphtate, 10% to 20% of a dimethylaminoethyl methacrylate-methacrylic acid ester copolymer, and 0.5% to 10% of a medium chain fatty acid ester in an alcoholic solvent containing practically no water.

7. A preparation according to any one of claims 1 to 5 for use as a medicament.

8. A preparation according to any one of claims 1 to 5 for use in the topical treatment of mycosis.

**Claims for the following Contracting States: ES, GR**

1. A process for the production of a film-forming antifungal preparation which comprises combining 0.1% to 1.5% of tolnaphtate, 10% to 20% of a dimethylaminoethyl methacrylate-methacrylic acid ester copolymer, and 0.5% to 10% of a medium chain fatty acid ester in an alcoholic solvent which contains practically no water.

2. The process claimed in Claim 1, wherein said medium chain fatty acid ester is tetraglyceryl monocaprate.

3. The process claimed in Claim 1, wherein the preparation further contains 0.1% to 5% of a thickening agent.

4. The process claimed in Claim 1, wherein said alcoholic solvent is ethanol or isopropanol.

5. The process claimed in Claim 3, wherein said thickening agent is ethyl cellulose, hydroxypropyl cellulose, or hydroxypropylmethyl cellulose.

**Revendications**
**Revendications pour les Etats contractants suivants : AT, BE CH, DE, FR,, GB, IT, LI, LU, NL, SE**

1. Préparation antifongique du type filmogène, qui comprend 0,1 % à 1,5 % de tolnaphtate, 10 % à 20 % d'un copolymère d'ester d'acide méthacrylique-diméthylaminoéthyl méthacrylate et 0,5 % à 10 % d'un ester d'acide gras à chaîne moyenne dans un solvant alcoolique mais ne contenant pratiquement pas d'eau.

2. Préparation antifongique du type filmogène suivant la revendication 1, caractérisée en ce que l'ester d'acide gras à chaîne moyenne est du monocaprate de tétraglycéryle.

3. Préparation antifongique du type filmogène suivant la revendication 1, caractérisée en ce qu'elle contient en outre 0,1 % à 5 % d'un agent épaississant.

4. Préparation antifongique du type filmogène suivant la revendication 1, caractérisée en ce que le solvant alcoolique est de l'éthanol ou de l'isopropanol.

5. Préparation antifongique du type filmogène suivant la revendication 3, caractérisée en ce que l'agent épaississant est de l'éthyl cellulose, de l'hydroxypropyl cellulose ou de l'hydroxypropylméthyl cellulose.

5

**6.** Procédé de préparation d'une préparation antifongique du type filmogène, qui comprend la combinaison de 0,1 % à 1,5 % de tolnaphtate, de 10 % à 20 % d'un copolymère d'ester d'acide méthacrylique-diméthylaminoéthyl méthacrylate et de 0,5 % à 10 % d'un ester d'acide gras à chaîne moyenne dans un solvant alcoolique ne contenant pratiquement pas d'eau.

**7.** Préparation suivant l'une quelconque des revendications 1 à 5, utilisable comme médicament.

**8.** Préparation suivant l'une quelconque des revendications 1 à 5, utilisable dans le traitement topique de la mycose.

**Revendications pour les Etats contractants suivants : ES, GR**

**1.** Procédé pour la production d'une préparation antifongique du type filmogène, qui comprend la combinaison de 0,1 % à 1,5 % de tolnaphtate, de 10 % à 20 % d'un copolymère d'ester d'acide méthacrylique-diméthylaminoéthyl méthacrylate et de 0,5 % à 10 % d'un ester d'acide gras à chaîne moyenne dans un solvant alcoolique qui ne contient pratiquement pas d'eau.

**2.** Procédé suivant la revendication 1, caractérisé en ce que l'ester d'acide gras à chaîne moyenne est du monocaprate de tétraglycéryle.

**3.** Procédé suivant la revendication 1, caractérise en ce que la préparation contient en outre 0,1 % à 5 % d'un agent épaississant.

**4.** Procédé suivant la revendication 1, caractérisé en ce que le solvant alcoolique est de l'éthanol ou de l'isopropanol.

**5.** Procédé suivant la revendication 3, caractérisé en ce que l'agent épaississant est de l'éthyl cellulose, de l'hydroxypropyl cellulose ou de l'hydroxypropylméthyl cellulose.

**Patentansprüche**
**Patentansprüche für folgende Vertragsstaaten : AT, BE, CH, DE, FR, GB, IT, LI, LU, NL, SE**

**1.** Filmbildendes antimykotisches Präparat, das folgende Bestandteile enthält:
0,1 bis 1,5 % Tolnaphthat, 10 bis 20 % Dimethylaminoethylmethacrylat-Methacrylsäureester-Copolymerisat und 0,5 bis 10 % eines Esters einer Fettsäure mittlerer Kettenlänge in einem alkoholischen Lösungsmittel, aber praktisch ohne einen Gehalt an Wasser.

**2.** Filmbildendes antimykotisches Präparat nach Anspruch 1, wobei es sich beim Ester einer Fettsäure mittlerer Kettenlänge um Tetraglycerylmonocaprat handelt.

**3.** Filmbildendes antimykotisches Präparat nach Anspruch 1, das zusätzlich 0,1 bis 5 % eines Verdikkungsmittels enthält.

**4.** Filmbildendes antimykotisches Präparat nach Anspruch 1, wobei es sich beim alkoholischen Lösungsmittel um Ethanol oder Isopropanol handelt.

**5.** Filmbildendes antimykotisches Präparat nach Anspruch 3, wobei es sich beim Verdickungsmittel um Ethylcellulose, Hydroxypropylcellulose oder Hydroxypropylmethylcellulose handelt.

**6.** Verfahren zur Herstellung eines filmbildenden antimykotischen Präparats umfassend die Vereinigung von 0,1 bis 1,5 % Tolnaphtat, 10 bis 20 % Dimethylaminoethylmethacrylat-Methacrylsäureester-Copolymerisat und 0,5 bis 10 % eines Esters einer Fettsäure mittlerer Kettenlänge in einem alkoholischen Lösungsmittel, das praktisch kein Wasser enthält.

**7.** Präparat nach einem der Ansprüche 1 bis 5 zur Verwendung als Arzneimittel.

**8.** Präparat nach einem der Ansprüche 1 bis 5 zur Verwendung bei der topischen Behandlung von Mykose.

**EP 0 319 964 B1**

**Patentansprüche für folgende Vertragsstaaten : ES, GR**

1. Verfahren zur Herstellung eines filmbildenden antimykotischen Präparats, umfassend die Vereinigung von 0,1 bis 1,5 % Tolnaphtat, 10 bis 20 % Dimethylaminoethylmethacrylat-Methacrylsäureester-Gopolymerisat und 0,5 bis 10 % eines Esters einer Fettsäure mittlerer Kettenlänge in einem alkoholischen Lösungsmittel, das praktisch kein Wasser enthält.

2. Verfahren nach Anspruch 1, wobei es sich bei dem Ester einer Fettsäure mittlerer Kettenlänge um Tetraglycerylmonocaprat handelt.

3. Verfahren nach Anspruch 1, wobei das Präparat zusätzlich 0,1 bis 5 % eines Verdickungsmittels enthält.

4. Verfahren nach Anspruch 1, wobei es sich beim alkoholischen Lösungsmittel um Ethanol oder Isopropanol handelt.

5. Verfahren nach Anspruch 3, wobei es sich beim Verdickungsmittel um Ethylcellulose, Hydroxypropylcellulose oder Hydroxypropylmethylcellulose handelt.

7

FIG. 1